# EUROPÄISCHE PATENTSCHRIFT

(11) **EP 1 461 459 B1**
(45) Veröffentlichungstag und Bekanntmachung des Hinweises auf die Patenterteilung: **27.09.2006**
(21) Anmeldenummer: 02796525.0
(22) Anmeldetag: 31.12.2002
(51) Int. Cl.: C12Q 1/68

(54) **VERFAHREN ZUR DETEKTION VON NUKLEINSÄUREN UNTER VERWENDUNG FESTPHASENGEBUNDENER PRIMER UND ABSPALTUNG DER PRODUKTE EINER ZYKLISCHEN AMPLIFIKATIONSREAKTION (TRAP RELEASE PRIMER AMPLIFICATION)**
METHODS FOR DETECTING NUCLEIC ACIDS WHILE USING SOLID PHASE BOUND PRIMERS AND CLEAVING THE PRODUCTS OF A CYCLIC AMPLIFICATION REACTION (TRAP RELEASE PRIMER AMPLIFICATION (TRAMP))
PROCEDE DE DETECTION D'ACIDES NUCLEIQUES FAISANT APPEL A DES AMORCES LIEES EN PHASE SOLIDE ET CONSISTANT A CLIVER LES PRODUITS ISSUS D'UNE REACTION D'AMPLIFICATION CYCLIQUE (TRAMP)

(30) Priorität: 31.12.2001 DE 10164219
(43) Veröffentlichungstag der Anmeldung: 29.09.2004
(73) Patentinhaber: Repp, Reinald, 36039 Fulda (DE)
(72) Erfinder: Repp, Reinald, 36039 Fulda (DE)
(74) Vertreter: Weiss, Wolfgang
(86) Internationale Anmeldenummer: PCT/DE2002/004754
(87) Internationale Veröffentlichungsnummer: WO 2003/057908

(56) Entgegenhaltungen:
- WO-A-00/22163
- DE-A- 19 624 562
- SHAPERO, MICHAEL H. ET AL: "SNP genotyping by multiplexed solid-phase amplification and fluorescent minisequencing" GENOME RESEARCH , Bd. 11, Nr. 11, 2001, Seiten 1926-1934, XP002252459
- ADESSI C ET AL: "Solid phase DNA amplification: characterization of primer attachment and amplification mechanisms" NUCLEIC ACIDS RESEARCH, OXFORD UNIVERSITY PRESS, SURREY, GB, Bd. 28, Nr. 20, 15. Oktober 2000 (2000-10-15), Seite e87 XP002169484 ISSN: 0305-1048 in der Anmeldung erwähnt

### SEQUENZPROTOKOLL

<110> Repp Prof., Reinald
<120> Verfahren zur Detektion von Nukleinsäuren unter Verwendung festphasengebundener Primer und Abspaltung der Produkte einer zyklischen Amplifikationsreaktion (Trap Release Primer Amplification (TRAmp))
<130> 29808PMO_RI
<140> PCT/DE02/04754
   <141> 2002-12-31
<150> DE10164219.9
   <151> 2001-12-31
<160> 1
<170> PatentIn Ver. 2.1
<210> 1
   <211> 32
   <212> DNA
   <213> Künstliche Sequenz
<220>
   <221> misc_binding
   <222> (1)
   <223> beliebige Sequenz
<220>
   <221> misc_binding
   <222> (32)
   <223> beliebige Sequenz
<220>
   <223> Beschreibung der künstlichen Sequenz:PCR-Primer
<400> 1
   ntttgtctcg agtgttttca ctcgagactt tn 32

## Beschreibung

Die vorliegende Erfindung betrifft Verfahren zur Detektion von Nukleinsäuren mit Hilfe einer Amplifikationsreaktion, wobei eine neuartige Kombination von festphasengebundenen Startermolekülen (Primem) mit zyklischer Amplifikationsreaktion an der Festphase und Überführung der Amplifikationsprodukte in die wässrige Phase zum Einsatz kommen. Durch die Kopplung der Primer an einer Festphase werden entscheidende Nachteile der klassischen Polymerase-Kettenreaktion (PCR), wie die Primer-Dimer-Bildung und die Entstehung falsch positiver Ergebnisse durch Produkt-Produkt-Interaktionen entscheidend reduziert. Um eine exponentielle Produktvermehrung zu erreichen, müssen jedoch die in jedem Zyklus neu synthetisierten PCR-Produkte von der Festphase freigesetzt werden. Dies wird durch eine Konformationsänderung innerhalb der Primer erreicht, die von der Bindung an die zu erkennende Zielsequenz ausgelöst wird und die selektive Abspaltung zum Beispiel durch ein Restriktionsenzym erlaubt.

Die Polymerase-Kettenreaktion (PCR) stellt ein Verfahren zur exponentiellen Vermehrung von spezifischen DNA-Abschnitten dar. Der allgemein dabei angewandte Reaktionsablauf ist sehr einfach, da alle Reagentien und Startermoleküle im Reaktionsgemisch vorliegen und die für den zyklischen Reaktionsablauf erforderlichen Absoluttemperaturen und Temperaturwechsel tolerieren. Auf diese Weise kann durch die sich vielfach, in der Regel 25 - 50fach wiederholende Abfolge von drei Schritten, der Denaturierung doppelsträngiger DNA-Moleküle zu Einzelsträngen, Bindung der passenden Einzelstränge an die komplementären Startermoleküle (Annealingphase) und der enzymatischen Verlängerung der Startermoleküle (Primer) entlang der gebundenen komplementären Zielsequenz (Elongation) eine Verfielfachung der Zielsequenz erreicht werden, woraus eine hohe Sensitivität resultiert. Unter optimalen Bedingungen werden die Moleküle der Zielsequenz in jedem dieser Zyklen verdoppelt Diese theoretische Effizienz wird jedoch in der Praxis höchstens während der ersten Reaktionszyklen erreicht (exponentielle Phase der PCR). In späteren Zyklen treten zunehmend sich negativ auswirkende Effekte auf, die die Effizienz der PCR-Amplifikation reduzieren und zu einer asymptotischen Annäherung der Zahl der Zielsequenzmoleküle an einen reaktionsspezifischen Grenzwert führen, über den hinaus keine weitere Vermehrung mehr erfolgt. Die Ursache hierfür liegt zum einen im zunehmenden Verbrauch der eingesetzten Ressourcen (Enzymaktivität, Primer, Nukleotidtriphosphate), zum größeren Teil jedoch an zunehmend auftretenden Wechselwirkungen zwischen den im Reaktionsansatz befindlichen Nukleinsäuren. Von Bedeutung sind hier beispielsweise unspezifische Primerbindungen, die zu einer linearen "Hintergrundamplifikation", oder sogar zu falsch positiven Ergebnissen führen können, wenn ein zweiter Primer am Gegenstrang benachbart bindet. Diese Effekte spielen besonders bei sogenannten multiplex-PCR-Reaktionen eine Rolle, bei denen eine größere Zahl von Primem zur Erfassung mehrerer Zielsequenzen in einem einzigen Reaktionsansatz eingesetzt wird. Ein weiteres Problem, das ebenfalls bei der multiplex-PCR an Bedeutung gewinnt ist die Bildung von Primer-Dimeren. Hierbei interagieren die eingesetzten Startermoleküle (Primer) miteinander, so daß es zu einer DNA-Kettenverlängerung entlang der zweiten Primersequenz kommt. Solche Primer-Dimere werden aufgrund ihrer geringen Größe sehr effizient amplifiziert, so daß die in der PCR eingesetzten Ressourcen bereits vor einer erkennbaren Amplifikation der Zielsequenz aufgebraucht sein können.
Nach Ablauf vieler PCR-Zyklen können zunehmend kompetitive Effekte zwischen den beiden Strängen des PCR-Produkts und den Primem die Amplifikationseffizienz beeinträchtigen. Die Amplifikation der Zielsequenz wird hierbei durch die vollständige oder partielle Paarung der beiden DNA-Stränge der Zielsequenz noch vor oder während der Kettenverlängerung beeinträchtigt. Neben der Beeinträchtigung der Amplifikationseffizienz kann die Wechselwirkung zwischen bereits in der PCR neu synthetisierten DNA-Molekülen zur Bildung unspezifischer Produkte, bzw. zu falsch posititiven Ergebnissen führen. Dies ist dann der Fall, wenn die 3'-Enden dieser DNA-Einzelstränge miteinander wechselwirken können. Auch hier ist die Gefahr bei multiplex-Reaktionen größer, da mit jeder zu amplifizierenden Zielsequenz die Gefahr steigt, daß DNA-Stränge mit zueinander homologen 3'-Enden entstehen, die dann nach Paarung aneinander und Vervollständigung zu DNA-Doppelsträngen durch die eingesetzte DNA-Polymerase in den folgenden Zyklen exponentiell amplifiziert werden können, was schließlich zur Entstehung falsch positiver bzw. artefizieller Produkte führt. Obwohl mehrere der beschriebenen, die PCR-Amplifikation beeinträchtigenden Mechanismen, sich bei multiplex-Reaktionen gravierender auswirken, werden multiplex-Reaktionen zunehmend eingesetzt, da sie es erlauben mit einer einzigen Reaktion gleich mehrere Zielsequenzen zu erfassen. Da sich die Zahl der bekannten Nukleinsäuresequenzen rasant vergrößert, ist zu erwarten, daß diese Tendenz anhält.
Als Alternative für die parallele Erfassung vieler Nukleinsäure-Zielsequenzen können auch sogenannte "Gene-chips" eingesetzt. Diese besitzen jedoch gegenüber der PCR eine viel geringere Sensitivität, da sie die Nukleinsäuren über spezifische Hybridisierungsreaktionen detektieren und es nicht zu einer enzymatischen Vermehrung der Zielsequenzen kommt.

Die der vorliegenden Erfindung zugrundeliegende Aufgabe besteht somit darin, die Zahl der in einer PCR-Reaktion parallel erfaßbaren Zielsequenzen zu erhöhen, in dem die oben beschriebenen bei multiplex-Reaktionen zunehmend auftretenden Störfaktoren, wie die Prüner-Dimer-Bildung und die Generierung falsch positiver Resultate als Folge unspezifischer Produkt-Produkt-Wechselwirkungen über komplementäre 3'-Enden weitgehend ausgeschaltet werden, aber gleichzeitig eine exponentielle Amplifikation der Zielsequenzen erfolgen kann.

Erfindungsgemäß wird diese Aufgabe gelöst durch ein Verfahren zur Amplifikation von Nukleinsäuren umfassend die Schritte:
i) Bereitstellen von zu amplifizierenden Nukleinsäuren,
ii) Durchführung der Amplifikationsreaktion unter Verwendung von mindestens 2 Primern, von denen mindestens einer an eine Festphase gekoppelt ist,
iii) Überführung der Reaktionsprodukte in die wässrige Phase,
iv) Detektieren der in ii) gebildeten Amplifikationsprodukte über fluoreszenzoptische, radioaktive, chemische, chromatographische oder jegliche andere Verfahren zur Detektion von Nukleinsäuren,
   dadurch gekennzeichnet, daß
   a) mindestens einer der für die Amplifikationsreaktion eingesetzten Primer in 5'-3'-Richtung folgende Elemente umfasst: Festphasenkopplungsstelle, einen ersten für die Zielsequenz spezifischen Abschnitt, eine doppelstrangspezifische Schnittstelle, eine fakultativ vorhandene Sequenz, die der Produktdetektion, Re-Amplifikation in einer nachgeschalteten konventionellen PCR-Reaktion (nested-PCR), als Primerbindungsstelle für eine Sequenzreaktion oder weiteren Funktionen dienen kann, fakultative weitere Sequenzabschnitte, die der Stabilisierung der doppelstrangspezifischen Schnittstelle dienen und eine zweite für die zu amplifizierende Nukleinsäure spezifische Sequenz,
   b) daß neu synthetisierte DNA-Moleküle noch während der Neusynthese oder nach den PCR-Amplifikationszyklen durch Abspaltung mit einem Restriktionsenzym in die wässrige Phase überführt werden.

Die Kopplung von PCR-Primem an eine Festphase kann zwar die Bildung von Primer-Dimeren verhindern, gleichzeitig ist jedoch eine exponentielle Produktvermehrung nicht mehr möglich, da nur die in der wässrigen Phase vor Start der Reaktion bereits vorliegenden Moleküle der Zielsequenz an die festphasengebundenen Primer hybridisieren und durch Verlängerung der Primer eine DNA-Neusynthese initiieren können. Da die neu synthetisierte DNA, die durch die Verlängerung der festphasengebundenen Primer entstanden ist, an der Festphase gebunden bleibt, kann sie nur äußerst begrenzt in unmittelbarer Nachbarschaft an komplementäre Primersequenzen binden und steht deshalb fast vollständig nicht als Matrize für eine weitere DNA-Synthese zur Verfügung. D.h. der Pool der eine DNA-Neusynthese ermöglichenden Matrizenstränge der Zielsequenz erweitert sich nicht wesentlich während der Reaktionszyklen, woraus nur eine lineare Amplifikationskinetik resultiert (Adessi et al. (2000), Nucleic Acids Research 28,20, e87).
Eine essentielle Voraussetzung für eine exponentielle Amplifikation der Zielsequenzen ist die Überführung der in der PCR synthetisierten DNA-Moleküle in den Pool potentieller DNA-Matrizenstränge, die bei Bindung komplementärer Primer wiederum eine DNA Neusynthese erlauben. Hierfür ist es erforderlich, daß beide DNA-Stränge der Zielsequenz nach jedem PCR-Zyklus frei mit komplementären Primern hybridisieren können. Dies kann erreicht werden, wenn die neu synthetisierte DNA in die wässrige Phase überführt wird, ohne die Bindung der einzelsträngigen Primer von der Festphase zu beeinträchtigen. Hierfür können Restriktionsenzyme verwendet werden, da diese selektiv doppelesträngige DNA an definierten Erkennungssequenzen schneiden. Wird die doppelsträngige DNA von der Festphase abgespalten, so können nach dem PCR-Denaturierungsschritt beide Stränge mit den komplementären Primern hybridisieren. Der Pool potentieller DNA-Matrizenstränge kann sich bei jedem Zyklus verdoppeln; eine exponentielle Amplifikation der Zielsequenz ist möglich.
Eine entscheidende Voraussetzung hierfür ist jedoch, daß die Erkennungssequenz für Restriktionsenzyme durch den Schnitt nicht zerstört wird. D.h. die verwendeten Enzyme müssen am 5'-Ende der Erkennungssequenz oder noch weiter 5'-wärts davor schneiden. Nur so bleibt die Erkennungssequenz erhalten und kann auch in folgenden Zyklen wieder eine Abspaltung doppelsträngiger DNA-Moleküle von der Festphase vermitteln. Eine Schnittstelle weiter 5'-wärts als direkt am 5'-Ende der Erkennungssequenz ist günstiger, da Restriktionsenzyme weniger effizient schneiden, wenn sich ihre Erkennungssequenz direkt am Ende eines doppelsträngigen DNA-Moleküls befindet und nicht noch einige weitere endständige Basenpaarungen vorliegen.

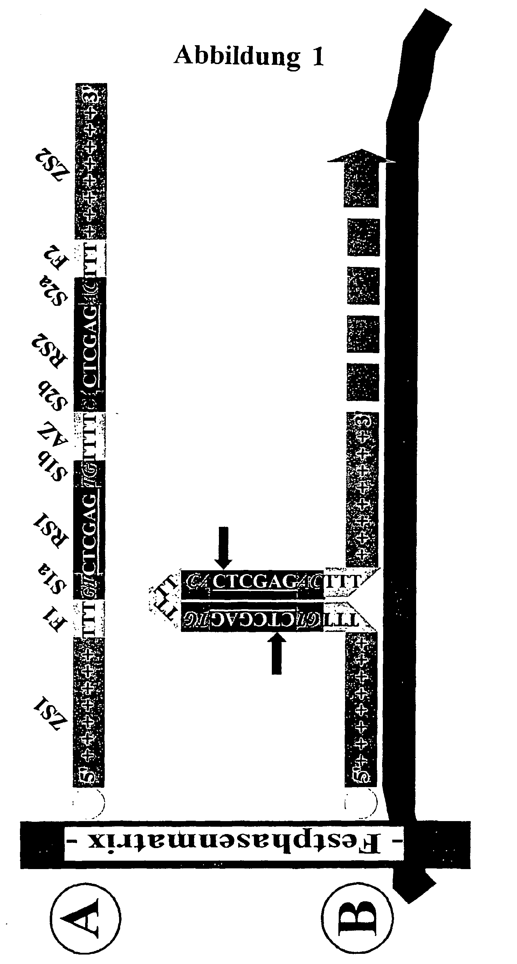

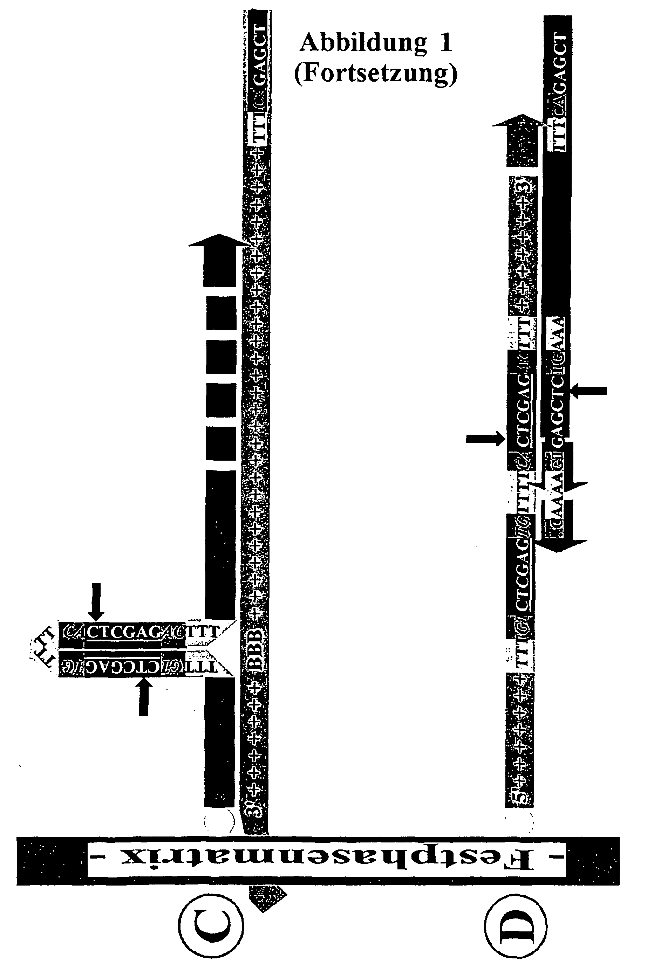

Die Durchführung der TR-PCR kann analog der konventionellen PCR erfolgen, wenn das verwendete Restriktionsenzym die in der PCR eingesetzten Temperaturen und Pufferbedingungen toleriert. Die Festphasenkopplung der PCR-Primer kann direkt oder indirekt an das Reaktionsgefäß selbst erfolgen einschließlich von Kunststoffgefäßen und Glaskapillaren oder die Primer werden an einen Träger gekoppelt, der dann in das Reaktionsgefäß eingebracht wird. Die entscheidenden Schritte des Reaktionsablaufs werden in Abbildung 1 schematisch dargestellt:

In Abschnitt "A" der Abbildung 1 sind die verschiedenen Domänen der PCR-Primer dargestellt. Am 5'-Ende befindet sich eine Struktur (als Kreis dargestellt), die die Bindung des Oligonukleotids an die Festphase vermittelt. Daran schließt sich in 3'-Richtung eine erste zur Zielsequenz komplementäre Domäne (ZS1) an. Diese wird in ihrer Länge und Sequenz auf die in der PCR-Reaktion verwendete "annealing" Temperatur abgestimmt. Bei dieser Temperatur soll die spezifische Bindung zwischen dem Primer und der zu amplifizierenden Nukleinsäure-Zielsequenz erfolgen. Eine analoge zur Zielsequenz bindende Domäne befindet sich am äußersten 3'-Ende der Primer (ZS2). Diese beiden an die Zielsequenz bindenden Primerdomänen werden durch eingefügte Nukleotide getrennt, die bei Bindung des Primers an die Zielsequenz durch Konformationsänderung eine doppelsträngige Restriktionsschnittstelle generieren. Von 5`- in 3'-Richtung umfaßt dieser Bereich die folgenden funktionellen Abschnitte: Flexibilisierungsdomäne 1 (F1, hier beispielsweise mit 3 "T"-Nukleotiden dargestellt), Stabilisierungsbereich 1a (S1a, kursive Nukleotidbezeichnungen), Schnittstellensequenz 1 (RS1, unterstrichene Nukelotidbezeichnungen), Stabilisierungsbereich 1b (S1b), Abstandshalter AZ (dieser zentrale Abstandshalter besteht optimalerweise aus 4 "T"-Nukleotiden, da diese eine vollständige Richtungsänderung innerhalb der dreidimensionalen Molekülstruktur vermitteln und so die Ausbildung einer Haarnadelstruktur erleichtern), Stabilisierungsdomäne 2b (S2b), Schnittstellensequenz 2 (RS2), Stabilisierungsdomäne 2a (S2a), Flexibilisierungsdomäne 2 (F2). Die Stabilisierungs- und Schnittstellendomänen 1 sind zu den analogen Domänen Nummer 2 in ihrer Nukleotidsequenz revers komplementär. Hierdurch kann sich bei Unterschreitung einer bestimmten Grenztemperatur eine Haarnadelstruktur innerhalb des Primers ausbilden. Neben der Schnittstellensequenz bestimmen Länge und Sequenz der Stabilisierungsdomänen diese Grenztemperatur. Sie wird so gewählt, daß sie unterhalb aller während des Ablaufs der PCR-Reaktion eingestellten Temperaturen liegt. Hierdurch wird gewährleistet, daß die festphasengebundenen Primer während der PCR-Reaktion in linearer Struktur vorliegen und keine Haamadelstrukturen ausbilden, solange die Primer nicht an die Zielsequenz mit ihren beiden endständigen spezifischen Domänen binden. Außer den beiden an die Zielsequenz bindenden Domänen (ZS1 und ZS2) und den Schnittstellensequenzen (RS1 und RS2) dienen alle anderen Primerabschnitte nur als Hilfsmittel zur Erzielung einer bestimmten Struktur in Abhängigkeit von der Bindung an die Zielsequenz und müssen somit nicht immer vorhanden sein.
Teil "B" der Abbildung stellt die Strukuren dar, wie sie nach Bindung eines Primers an seine Zielsequenz entstehen ("- - -" soll die im Beispiel gewählte negative Polarität symbolisieren). Durch Bindung der beiden spezifischen Primerdomänen an die Zielsequenz werden diese beiden Primerabschnitte in unmittelbare Nachbarschaft gebracht. Hierdurch wird die potentielle Haamadelstruktur, die der Primerbereich zwischen den beiden endständigen zur Zielsequenz spezifischen Domänen ausbilden kann, stabilisiert. Dies hat zur Folge, daß die Haarnadelstruktur nach Bindung des Primers an seine Zielsequenz auch bei höheren Temperaturen ausgebildet werden kann, als dies im einzelsträngigen Primermolekül der Fall ist. Durch entsprechende Sequenzauswahl der als "Stabilisierungsdomänen" bezeichneten Primerabschnitte und anderer sich auf die Schmelztemperatur der Haarnadelstruktur auswirkender Parameter, wie z.B. des Abstands zwischen den beiden Primerbindungsdomänen auf der Zielsequenz, wird die Grenztemperatur zwischen linearer Primerstruktur im nativen Zustand und Ausbildung einer Haarnadelstruktur nach Bindung des Primers an seine Zielsequenz so eingestellt, daß sie während des niedrigsten Temperaturniveaus der PCR-Reaktion geringfügig überschritten wird. D.h. nur Primer, die an ihre Zieslequenz gebunden sind, bilden während der PCR-Reaktion eine Haarnadelstruktur aus; einzelsträngige Primermoleküle bleiben dagegen während der gesamten PCR-Reaktion linear.
Der in der Zielsequenz als "BBB" dargestellte Abschnitt muß die Distanz zwischen den beiden Strängen der Haarnadelstruktur überbrücken, entsprechend dem Abstand, der zur Ausbildung der Wasserstoffbrückenbindungen zwischen den an der Haarnadelstruktur beteiligten Nukleotiden benötigt wird. Hier sollten in der Zielsequenz keine Nukleotide vorliegen, die mit den "Flexibilisierungsdomänen" der Primer (F1, F2) wechselwirken können. Da für die "Flexibilisierungsdomänen" in diesem Beispiel "T"-Nukleotide gewählt wurden, sollten in dem Abstandshalter (BBB) der Zielsequenz keine "A"-Nukleotide vorkommen. Durch Ausbildung der Haarnadelstruktur lagern sich die beiden zueinander revers komplementären Schnittstellendomänen des PCR-Primers zum DNA-Doppelstrang aneinander, so daß das entsprechende Restriktionsenzym schneiden kann (Schnittstellen sind durch schwarze Pfeile markiert). Wird ein hitzestabiles Restriktionsenzym verwendet, das auch unter den PCR-Puffer- und Temperaturbedingungen eine ausreichende Aktivität besitzt, so kann dieses dem PCR-Reaktionsgemisch einfach zugesetzt werden. In der Abbildung ist die Sequenz des hitzestabilen Restriktionsenzyms TliI dargestellt. Die festphasengebundenen Primer werden in ihrer linearer einzelsträngigen Struktur, die vorliegt solange keine Bindung an die Zielsequenz erfolgt ist, nicht geschnitten. Ist jedoch eine Bindung des Primers an seine Zielsequenz erfolgt, so bildet sich über die Entstehung einer "Haarnadelkonformation" die doppelsträngige Erkennungsdomäne für das entsprechende Restriktionsenzym aus. Der Primer wird in seinem zentralen Abschnitt von dem Restriktionsenzym gespalten, während die Strangverlängerung am 3'-Ende des Primers entlang der Zielsequenz erfolgt. Hierdurch wird der neu synthetisierte DNA-Strang zusammen mit dem 3`-Bereich des PCR-Primers von der Festphasenkopplungsstelle abgespalten. In der folgenden Denaturierungsphase der PCR, in der die Wasserstoffbrückenbindungen der DNA-Doppelstränge in hoher Temperatur getrennt werden, werden alle neu synthetisierten DNA-Einzelstränge freigesetzt und können im folgenden PCR-Zyklus wieder an komplementären Primersequenzen binden. Dies ist in Abschnitt "C" dargestellt.
Der in Abschnitt "C" dargestellte plussträngige Matrizenstrang enthält als Produkt des ersten Amplifilcationszyklus an seinem 5'-Ende Sequenzabschnitte, die von einem PCR-Primer stammen und Teile der Restriktionsschnittstelle beinhalten. Bis zu diesen Basen wird der komplementäre Minusstrang der Zielsequenz in diesem Beispielzyklus zum Doppelstrang vervollständigt. Wichtig ist es hierbei, daß in der PCR eine DNA-Polymerase verwendet wird, die keine zusätzlichen Nukleotide, meist einen Adenosinrest, am 3'-Ende des neu synthetisierten Strangs anfügt, es sei denn, genau diese Base entspricht der zur Vervollständigung der Restriktionsschnittstelle erforderlichen Sequenz.
In Abschnitt "D" ist beispielhaft dargestellt, wie der in Abschnitt "C" synthetisierte Minusstrang wiederum als Matrize für die erneute Plusstrangsynthese dienen kann. Der Minusstrang enthält bereits an beiden Enden von PCR-Primern stammende Sequenzabschnitte. Nach Bindung an die zielsequenzspezifische Domäne am 3'-Ende eines plussträngigen PCR-Primers kann sein eigenes 3'-Ende entlang der Primersequenz verlängert werden (nach links gerichtete Pfeile), während gleichzeitig die Synthese des neuen Plusstranges stattfindet (Pfeil nach rechts). Hierdurch wird die zuvor nur partiell vorhandene Restriktionschnittstelle regeneriert. Das Produkt dieses PCR-Laufs kann auch ohne die Ausbildung einer Haarnadelstruktur innerhalb des Primers abgespalten werden. Die Ausbildung einer Restriktionsschnittstelle innerhalb der PCR-Primer durch Ausbildung einer Haarnadelstruktur infolge spezifischer Bindung an die Zielsequenz stellt somit sicher, daß eine Abspaltung der neu synthetisierten PCR-Produkte auch ablaufen kann, sofern die Neusynthese an der nativen Ausgangs-DNA (Abschnitt "B") oder am Produkt einer ersten Amplifikationsreaktion erfolgt (Abschnitt "C"). Alle weiteren Amplifikationsprodukte enthalten an beiden Enden bereits von PCR-Primem stammende Teilsequenzen, die linear zu vollständigen Restriktionsschnittstellen verlängert werden können (Abschnitt "D").

Der dargestellte Reaktionsablauf setzt voraus, daß die zu amplifizierende Zielsequenz nicht die gleichen Restriktionsschnittstellen enthält, wie sie innerhalb der PCR-Primer verwendet werden. Durch Verwendung selten schneidender Restriktionsenzyme treten hierdurch keine wesentlichen Beschränkungen auf

Die Trap Release Primer Amplifikation (TRAmp) erlaubt es in multiplex-PCR Ansätzen die Zahl der gleichzeitig detektierbaren Zielsequenzen zu erhöhen, da unerwünschte Primer-Primer-Wechselwirkungen weitgehend ausgeschaltet werden. Die zur Amplifikation und Freisetzung der Produkte zumindest während der ersten Amplifikationszyklen erforderliche Bindung an 2 spezifische Sequenzabschnitte eines jeden Primers erhöht die Spezifität der Reaktion. Weiterhin kann eine große Zahl von PCR-Primern vorgefertigt an eine Festphase gekoppelt werden. Werden die Primer dabei an ein Reaktionsgefäß oder eine zur Reaktion zuzugebende Matrize gekoppelt, so werden multiplex-Reaktionen wesentlich vereinfacht, da die zur Zusammenstellung der Primerkombinationen erforderlichen Pipettierschritte entfallen.

## Patentansprüche

1. Verfahren zur Detektion von Nukleinsäuren unter Verwendung festphasengebundener Primer, die durch Bindung an ihre spezifische Zielsequenz eine Konformationsänderung erfahren, die die selektive Abspaltung der Produkte einer zyklischen Amplifikationsreaktion ermöglicht (Trap Release Primer Amplification (TRAmp)), umfassend die Schritte
i) Bereitstellen von zu amplifizierenden Nukleinsäuren,
ii) Durchführung der Amplifikationsreaktion unter Verwendung von mindestens 2 Primern, von denen mindestens einer an eine Festphase gekoppelt ist,
iii) Generierung einer Spaltstelle innerhalb der festphasengebundenen Primer durch eine Konformationsänderung infolge der Bindung an die primerspezifische Zielsequenz,
iv) Freisetzung der Reaktionsprodukte in die wässrige Phase durch Spaltung innerhalb der durch Konformationsänderung in iii) generierten Spaltstelle z.B. mit Hilfe eines Restriktionsenzyms,
v) Detektieren der in ii) gebildeten Amplifikationsprodukte über fluoreszenzoptische, radioaktive, chemische, chromatographische oder jegliche andere Verfahren zur Detektion von Nukleinsäuren,
**dadurch gekennzeichnet, daß**
a) mindestens einer der für die Amplifikationsreaktion eingesetzten Primer in 5'-3'-Richtung folgende Elemente umfasst: Festphasenkopplungsstelle, einen ersten für die Zielsequenz spezifischen Abschnitt, eine durch Konformationsänderung generierbare Schnittstelle, eine fakultativ vorhandene Sequenz, die der Produktdetektion, Re-Amplifikation in einer nachgeschalteten konventionellen PCR-Reaktion (nested-PCR), als Primerbindungsstelle für eine Sequenzreaktion oder weiteren Funktionen dienen kann, fakultative weitere Sequenzabschnitte, die der Stabilisierung der doppelstrangspezifischen Schnittstelle dienen und eine zweite für die zu amplifizierende Nukleinsäure spezifische Sequenz,
b) daß neu synthetisierte DNA-Moleküle noch während der Neusynthese oder nach den PCR-Amplifikationszyklen durch Abspaltung mit einem Restriktionsenzym in die wässrige Phase überführt werden.

## Claims

1. Method of detecting nucleic acids using solid-phase-bound primers which, by binding to their specific target sequence, undergo a conformation change which enables the selective cleavage of the products of the cyclic amplification reaction (trap release primer amplification (TRAmp)), comprising the steps
i) providing nucleic acids to be amplified,
ii) carrying out the amplification reaction using at least two primers, of which at least one is coupled with a solid phase,
iii) generating a cleavage site within the solid-phase-bound primers by a conformation change resulting from binding to the primer-specific target sequence,
iv) releasing the reaction products into the aqueous phase by cleavage within the cleavage site generated in ii) by the conformation change, e.g. with the aid of a restriction enzyme,
v) detecting the amplification products formed in ii) via fluorescent-optical, radioactive, chemical, chromatographic or any other methods for detecting nucleic acids,
**characterized in that**
a) at least one of the primers employed for the amplification reaction comprises, in 5'-3' direction, the following elements: coupling site for the solid phase, a first target-sequence-specific segment, a double-strand-specific cleavage site, an optional sequence which may serve for product detection, reamplification in subsequent conventional PCR reaction (nested PCR), as primer binding site for a sequence reaction or for further functions, optional further sequence segments which serve for the stabilization of the double-strand-specific cleavage site, and a second sequence which is specific for the nucleic acid to be amplified,
b) newly-synthesized DNA molecules are transferred into the aqueous phase by cleavage with the restriction enzyme either still during the synthesis phase or after the PCR amplification cycles.

## Revendications

1. Procédé pour la détection d'acide nucléique moyennant l'utilisation d'amorces liées sur phase solide qui, par liaison à leur séquence cible spécifique, subissent un changement de conformation qui permet l'élimination sélective des produits d'une réaction d'amplification cyclique (Trap Release Primer Amplification (TRAmp)), comprenant les étapes
i) mise à disposition d'acides nucléiques à amplifier,
ii) exécution de la réaction d'amplification moyennant l'utilisation d'au moins 2 amorces, dont au moins une est couplée à une phase solide
iii) génération d'un emplacement de clivage à l'intérieur des amorces liées à la phase solide par un changement de conformation par suite de la liaison à la séquence cible spécifique de l'amorce,
iv) libération des produits de réaction dans la phase aqueuse par clivage à l'intérieur de l'emplacement de clivage engendré en iii) par changement de conformation, par exemple à l'aide d'une enzyme de restriction,
v) détection des produits d'amplification formées en ii) par des procédés optiques à fluorescence, des procédés de radioactivité, des procédés chimiques, des procédés chromatographiques ou tous autres procédés pour la détection d'acides nucléiques,
**caractérisé en ce que**
a) au moins une des amorces utilisées pour la réaction d'amplification comprend dans la direction 5'-3' les éléments suivants : un emplacement de couplage à la phase solide, un premier tronçon spécifique pour la séquence cible, un emplacement de clivage pouvant être engendré par changement de conformation, une séquence présente facultativement qui peut servir à la détection du produit, à la ré-amplification dans une réaction PCR conventionnelle en aval (nested PCR), comme emplacement de liaison de l'amorce pour une réaction de séquence ou pour d'autres fonctions, d'autres tronçons de séquence facultatifs qui servent à la stabilisation de l'emplacement de clivage spécifique à la double chaîne et une deuxième séquence spécifique pour l'acide nucléique à amplifier,
b) encore pendant la synthèse à neuf ou après les cycles d'amplification PCR, des molécules d'ADN nouvellement synthétisées sont transférées dans la phase aqueuse par clivage avec une enzyme de restriction.
